(19) Europäisches Patentamt
European Patent Office
Office européen des brevets

(11) **EP 1 790 668 A1**

(12) **EUROPEAN PATENT APPLICATION**
published in accordance with Art. 158(3) EPC

(43) Date of publication:
**30.05.2007 Bulletin 2007/22**

(51) Int Cl.:
**C08F 6/00** (2006.01)

(21) Application number: **05753460.4**

(22) Date of filing: **22.06.2005**

(86) International application number:
**PCT/JP2005/011440**

(87) International publication number:
**WO 2006/008905 (26.01.2006 Gazette 2006/04)**

(84) Designated Contracting States:
**BE DE FR**

(30) Priority: **15.07.2004 JP 2004208310**

(71) Applicant: **SUMITOMO SEIKA CHEMICALS CO., LTD.**
**Kako-gun, Hyogo 675-0145 (JP)**

(72) Inventors:
• **MATSUDA, Kenya,**
**SUMITOMO SEIKA CHEMICALS CO.LTD**
**Himeji-shi, Hyogo 672-8076 (JP)**

• **KAKIMOTO, Hidenobu,**
**SUMITOMO SEIKA CHEM. CO. LTD**
**Himeji-shi, Hyogo 672-8076 (JP)**

• **NAWATA, Yasuhiro,**
**SUMITOMO SEIKA CHEMICALS CO. LTD**
**Himeji-shi, Hyogo 672-8076 (JP)**

(74) Representative: **HOFFMANN EITLE**
**Patent- und Rechtsanwälte**
**Arabellastrasse 4**
**81925 München (DE)**

(54) **METHOD FOR PRODUCING WATER-ABSORBING RESIN**

(57) The present invention provides a process for producing a water-absorbing resin with less coloring wherein only a small amount of a polymerization inhibitor remains, and the water-absorbing resin. More particularly, the present invention provides a process for producing a water-absorbing resin, comprising polymerizing a water-soluble ethylenic unsaturated monomer and, then, drying a hydrous gel of the resulting polymer at a pressure of 5 to 90 kPa under the atmosphere having no oxygen, or having an oxygen concentration of not higher than 5% by volume. In addition, by such the process, a water-absorbing resin having Yellow Index of not more than 20 is obtained, wherein an amount of a remaining polymerization inhibitor is not more than 30 ppm.

EP 1 790 668 A1

## Description

Technical Field

The present invention relates to a process for producing a water-absorbing resin. More particularly, the present invention relates to a process for producing a water-absorbing resin with less coloring, wherein only a small amount of a polymerization inhibitor remains.

Background Art

In recent years, a water-absorbing resin has been widely used in various fields such as a hygiene material such as a paper diaper, a sanitary material and the like, a horticultural material such as a water-retaining material, a soil improver and the like, and an industrial material such as a water shutting off material for a cable, a dew condensation preventing material and the like.

As the water-absorbing resin, a hydrolysate of a starch-acrylonitrile graft copolymer, a neutralized starch-acrylic acid graft copolymer, a saponified vinyl acetate-acrylic acid ester copolymer, partially neutralized polyacrylic acid and the like are known. In particular, partially neutralized polyacrylic acid is preferably used in a hygiene material or the like due to excellent productivity and economical property.

Meanwhile, a quinone compound as a polymerization inhibitor is added to a water-soluble ethylenic unsaturated monomer such as acrylic acid constituting partially neutralized polyacrylic acid, in order to maintain stability during transportation or at storage. For example about 200 ppm of p-methoxyphenol is added to acrylic acid. However, the quinone compound has a problem that it is easily changed to a coloring substance and the resulting water-absorbing resin is colored.

Then, as a method of reducing this polymerization inhibitor, a method of reducing a content of the polymerization inhibitor by mixing an acrylic acid-based polymer after polymerization with an oxidizing agent, or a method of reducing a content by contacting with an adsorbing agent has been proposed (see Patent Literature 1).

However, reduction in a polymerization inhibitor according to the above method separately requires a step for addition of an oxidizing agent, or addition and removal of an adsorbing agent, and it is difficult to say that the method is an industrially advantageous process.

Patent Literature 1: JP-A No. 2003-48915

Disclosure of the Invention

Problems to be solved by the Invention

An object of the present invention is to provide a process for easily producing a water-absorbing resin with less coloring, wherein a small amount of a polymerization inhibitor remains in the water-absorbing resin.

Means to Solve the Problem

That is, the present invention relates to a process for producing a water-absorbing resin, comprising polymerizing a water-soluble ethylenic unsaturated monomer and, then, drying a hydrous gel of the resulting polymer under the atmosphere containing no oxygen, or having an oxygen concentration of 5% by volume or lower at a pressure of 5 to 90 kPa.

Effect of the Invention

According to the present invention, a water-absorbing resin with less coloring, wherein a small amount of a polymerization inhibitor remains in the water-absorbing resin, can be easily produced.

Examples of the water-soluble ethylenic unsaturated monomer used in the present invention include (meth) acrylic acid ["(meth)acryl" means "acryl" or "methacryl"; the same hereinafter], 2-(meth)acrylamido-2-methylpropanesulfonic acid or an alkali metal salt thereof; a nonionic monomer such as (meth)acrylamide, N,N-dimethylacrylamide, 2-hydroxyethyl (meth)acrylate, N-methylol(meth)acrylamide etc.; an amino group-containing unsaturated monomer such as diethylaminoethyl (meth)acrylate, diethylaminopropyl (meth)acrylate etc., or a quaternized compound thereof, and they may be used alone, or may be used by mixing two or more kinds. Examples of an alkali metal in the alkali metal salt include lithium, sodium, potassium and the like.

Among the water-soluble ethylenic unsaturated monomer, preferable are (meth)acrylic acid or an alkali metal salt thereof, (meth)acrylamide and N,N-dimethylacrylamide which are industrially easily available.

The water-soluble ethylenic unsaturated monomer can be usually used as an aqueous solution. A concentration of a water-soluble ethylenic unsaturated monomer in an aqueous solution of the water-soluble ethylenic unsaturated monomer is preferably from 25% by weight to a saturated concentration.

When a water-soluble ethylenic unsaturated monomer used contains an acid group, the acid group may be neutralized with an alkali metal. A degree of neutralization with an alkali metal is preferably in a range of 10 to 100 mole % of an acid group of a water-soluble ethylenic unsaturated monomer before neutralization, from a viewpoint that an osmotic pressure of the resulting water-soluble resin is great, a water-absorbing rate is high, and a problem of safety due to the presence of an excessive alkali metal is not arisen. Examples of the alkali metal include lithium, sodium, potassium and the like. Among them, sodium and potassium are preferable.

A method of polymerizing a water-soluble ethylenic unsaturated monomer is not particularly limited, but a reverse phase suspension polymerization method, an aqueous solution polymerization method and the like which are a representative polymerization method are used. In the aqueous solution polymerization method, polymerization is performed by heating while an aqueous water-soluble ethylenic unsaturated monomer solution, a crosslinking agent and a water-soluble radical polymerization initiator are stirred, if necessary. In addition, in the reverse phase suspension polymerization method, polymerization is performed by heating an aqueous water-soluble ethylenic unsaturated monomer solution, a surfactant and/or a polymer protective colloid, a crosslinking agent as well as a water-soluble radical polymerization initiator in a hydrocarbon-based solvent under stirring.

A reverse phase suspension polymerization method as one example of embodiments of the present invention will be explained in more detail below.

Examples of the surfactant used in the reverse phase suspension polymerization method include nonionic surfactants such as sorbitan fatty acid ester, (poly)glycerin fatty acid ester ["(poly)" means both of the case where there is a prefix of "poly" and the case where there is no prefix of "poly"; the same hereinafter], sucrose fatty acid ester, sorbitol fatty acid ester, polyoxyethylene alkyl phenyl ether, hexaglyceryl monobeherate etc.; anionic surfactants such as fatty acid salt, alkylbenzenesulfonate salt, alkylmethyl taurate salt, polyoxyethylene alkyl phenyl ether sulfate ester salt, polyoxyethylene alkyl ether sulfonate salt etc., and they may be used alone, or may be used by mixing two or more kinds. Among them, sorbitan fatty acid ester, polyglycerin fatty acid ester, and sucrose fatty acid ester are preferable.

In addition to the surfactants, a polymer protective colloid may be used together. Examples of the polymer protective colloid include ethylcellulose, ethylhydroxyethylcellulose, polyethylene oxide, anhydrous maleicized polyethylene, anhydrous maleicized polybutadiene, anhydrous maleicized EMDM (ethylene/propylene/diene/terpolymer) and the like, and they may be used alone, or may be used by mixing two or more kinds.

An amount of the surfactant and/or the polymer protective colloid is preferably 0.05 to 5 parts by weight, more preferably 0.1 to 3 parts by weight based on 100 parts by weight of an aqueous solution of the water-soluble ethylenic unsaturated monomer.

Examples of the water-soluble radical polymerization initiator include persulfate salts such as potassium persulfate, ammonium persulfate, sodium persulfate etc.; azo compounds such as 2,2'-azobis(2-amidinopropane) dihydrochloride, azobis(cyanovaleric acid) etc., and they may be used alone, or may be used by mixing two or more kinds. Alternatively, a radical polymerization initiator may be used as a redox polymerization initiator by using a sulfite salt or the like. Among them, potassium persulfate, ammonium persulfate and sodium persulfate are preferable from a viewpoint that they are easily available and have better storage stability.

An amount of the radical polymerization initiator is preferably 0.00001 to 0.02 mole, more preferably 0.0001 to 0.01 mole per 1 mole of a water-soluble ethylenic unsaturated monomer from a viewpoint that a time for a polymerization reaction is shortened, and a rapid polymerization reaction is prevented.

Examples of the hydrocarbon-based solvent include aliphatic hydrocarbons such as n-hexane, n-heptane, ligroin etc.; alicyclic hydrocarbons such as cyclopentane, methylcyclopentane, cyclohexane, methylcyclohexane etc.; aromatic hydrocarbons such as benzene, toluene, xylene etc., and they may be used alone, or may be used by mixing two or more kinds. Among them, n-hexane, n-heptane, and cyclohexane are preferable from a viewpoint that they are industrially easily available and have stable quality and the low cost.

An amount of the hydrocarbon-based solvent is preferably 50 to 600 parts by weight, more preferably 100 to 550 parts by weight based on 100 parts by weight of a water-soluble ethylenic unsaturated monomer from a viewpoint that polymerization heat is removed, thereby, a polymerization temperature is easily controlled.

Examples of the crosslinking agent include diols, triols or polyols such as (poly)ethylene glycol ["(poly)" means both of the case where there is a prefix of "poly" and the case where there is no prefix of "poly"], (poly)propylene glycol, 1,4-butanediol, trimethylol propane, (poly)glycerin etc.; unsaturated polyesters obtained by reacting the diols, triols or polyols with an unsaturated acid such as (meth)acrylic acid, maleic acid, fumaric acid etc.; bisacrylamides such as N, N'-methylenebisacrylamide etc.; di- or tri(meth)acrylic acid esters obtained by reacting polyepoxide and (meth)acrylic acid; di(meth)acrylic acid carbamyl esters obtained by reacting polyisocyanate such as tolylene diisocyanate, hexamethylene diisocyanate and the like with hydroxyethyl(meth)acrylate; compounds having two or more polymerizable unsaturated groups such as diallylated starch, diallylated cellulose, diallyl phthalate, N,N',N''-triallyl isocyanate, divinylbenzene etc.; diglycidyl ether compounds such as (poly)ethylene glycol diglycidyl ether, (poly)propylene glycol diglycidyl ether, (poly)glycerin diglycidyl ether etc.; haloepoxy compounds such as epichlorohydrin, epibromohydrin, α-methylepichlorohydrin etc.; compounds having two or more reactive functional groups such as isocyanate compounds such as 2,4-tolylene diisocyanate, hexamethylene diisocyanate etc.; oxetane compounds such as 3-methyl-3-oxetanemethanol, 3-ethyl-3-oxetanemethanol, 3-butyl-3-oxetanemethanol, 3-methyl-3-oxetanethanol, 3-ethyl-3-oxetanethanol, 3-butyl-3-oxetanethanol etc., and they may be used alone, or may be used by mixing two or more kinds. Among them, ethylene glycol diglycidyl ether, propylene glycol diglycidyl ether, glycerin diglycidyl ether, polyethylene glycol diglycidyl ether, polypropylene glycol diglycidyl ether, polyglycerin diglycidyl ether and N,N'-methylenebisacrylamide are preferable due to excellent reactivity at a low temperature.

An amount of the crosslinking agent is preferably not more than 3 parts by weight, more preferably 0.001 to 1 part by weight based on 100 parts by weight of a water-soluble ethylenic unsaturated monomer from a viewpoint that a water-soluble nature of the resulting polymer is suppressed by suitable crosslinking, and the polymer exhibits sufficient water-absorbing property.

Since a reaction temperature upon polymerization is different depending on a kind of a water-soluble radical polymerization initiator, it can not be indiscriminately determined. Usually, the reaction temperature is preferably 20 to 110 °C, more preferably 40 to 90 °C from a viewpoint that polymerization proceeds rapidly, a polymerization time is shortened, the temperature is economically preferable, polymerization heat is easily removed, and a reaction is performed smoothly. A reaction time is usually 0.1 to 4 hours.

A reaction solution containing the thus obtained polymer is a solution in which a hydrated polymer is dispersed in a mixed solvent of the hydrocarbon-based solvent and water. A hydrocarbon-based solvent is distilled off from a reaction solution containing the polymer by heating to a temperature of not lower than a boiling point of the hydrocarbon-based solvent, to obtain a hydrous gel of a polymer.

By drying the resulting hydrous gel under the atmosphere containing no oxygen, or having a specified oxygen concentration or lower at a specified pressure, a water-absorbing resin with less coloring, wherein a small amount of a polymerization inhibitor remains in the water-absorbing resin, can be produced.

The oxygen concentration is not higher than 5% by volume, preferably not higher than 3% by volume, further preferably not higher than 2% by volume. When the oxygen concentration exceeds 5% by volume, a polymerization inhibitor undergoes influence of oxygen, and is easily changed to a coloring substance, and the resulting water-absorbing resin becomes easy to be colored.

A component other than oxygen under the atmosphere having a specified oxygen concentration or lower is not particularly limited, but examples include nitrogen, helium, neon, argon and the like. Inter alia, nitrogen is preferable from a viewpoint of economical property.

A method of realizing the atmosphere at the aforementioned oxygen concentration is not particularly limited, but examples include a method of introducing a gas having a predetermined oxygen concentration into the interior of a dryer through a tube mounted in the interior of the dryer, and a method of pre-mixing a gas not containing oxygen and the air to a predetermined oxygen concentration and, similarly, introducing the mixture into the interior of a dryer. By filling a port for introducing a hydrous gel, a port for draining a dried water-absorbing resin, and a bearing part of a stirring axis of a dryer, into each of which the external air is easily leaked therein, with a gas of a predetermined oxygen concentration, the atmosphere of a predetermined oxygen concentration can be further easily realized.

A pressure upon drying is preferably 5 to 90 kPa, more preferably 10 to 60 kPa. When the pressure is lower than 5 kPa, a drying facility which can stand high vacuum becomes expensive, the economical effect is not obtained, and this is not preferable. On the other hand, when the pressure exceeds 90 kPa, the effect of reducing a polymerization inhibitor from a hydrous gel is deteriorated, and a drying time is lengthened, being not preferable.

A temperature upon drying is not particularly limited, but is preferably 60 °C to 110 °C, more preferably 60 °C to 90 °C from a viewpoint that degradation of a water-absorbing resin due to heat is suppressed, and a drying time is further shortened.

An end point of drying is a point at which a water content of a water-absorbing resin becomes 10 % by weight or less. A water content of a water-absorbing resin can be calculated according to the following equation:

$$\text{water content (\%)} = (\text{weight before drying} - \text{weight after drying}) \div \text{weight before drying} \times 100$$

by placing a water-absorbing resin into a hot air dryer at 105 °C , allowing to stand for 2 hours to dry it, and measuring weights before and after drying. A drying time is usually 0.5 to 5 hours.

A dryer used in the drying is not particularly limited, but for example, dryers which are generally used, such as a band-type dryer equipped with a pressure-reducing apparatus, a groove-type dryer, a rotation dryer, and a kneader equipped with a pressure-reducing device and a drying device can be used.

In the thus obtained water-absorbing resin, an amount of a remaining polymerization inhibitor is not more than 30 ppm, preferably not more than 20 ppm. When the amount of a remaining polymerization inhibitor exceeds 30 ppm, a water-absorbing resin becomes easy to be colored at drying, and this is not preferable.

On the other hand, Yellow Index of the resulting water-absorbing resin is not more than 20, preferably not more than 15. When Yellow Index exceeds 20, since it is seen that the resin is clearly colored even visually, when used in a hygiene material or the like, fine sight is greatly deteriorated, and a value as a merchandize is remarkably reduced.

An additive such as a lubricant, a deodorant, an antibacterial agent and the like may be further added to the water-absorbing resin obtained by the present invention, depending on the purposes. An amount of the additive is different depending on utility of a water-absorbing resin, a kind of an additive or the like, and is preferably 0.001 to 10 parts by weight, more preferably 0.01 to 5 parts by weight based on a total amount of 100 parts by weight of a water-soluble ethylenic unsaturated monomer which has been subjected to polymerization.

Examples

The present invention will be specifically explained by way of Preparation Examples, Examples, and Comparative Examples, but the present invention is not limited at all by these Examples.

Preparation Example 1

Into an Erlenmeyer flask having an inner volume of 500 ml was placed 92 g (1.02 mol) of 80% by weight of an aqueous acrylic acid solution, 159.3g of 19.3% by weight of an aqueous sodium hydroxide solution was added dropwise while ice-cooling to perform neutralization of 75 mol% of acrylic acid, thereby, 36% by weight of an aqueous acrylic acid partially neutralized salt solution was prepared. To the resulting aqueous acrylic acid partially neutralized salt solution were added 18.4 mg (0.12 mmol) of N,N'-methylenebisacrylamide and 92 mg (0.34 mmol) of potassium persulfate, and this was used as an aqueous monomer solution (a1) for first-stage polymerization.

On the other hand, to a five-necked cylindrical round-bottom flask of an inner volume of 2 liter, equipped with a stirrer, a two-step paddle wing, a refluxing condenser, an addition funnel and a nitrogen gas introducing tube were added 340 g (500 ml) of n-heptane and 0.92 g of polyglycerin fatty acid ester (trade name of Taiyo Kagaku Co., Ltd.: SUNSOFT Q-185S) to dissolve the material in n-heptane, and the aqueous monomer solution (a1) for polymerization was added, and the mixture was suspended under stirring. Thereafter, the system was replaced with nitrogen, a temperature was raised to 70 °C, and first-stage reverse phase suspension polymerization was performed.
Then, separately, 92 g (1.02 mol) of 80% by weight of an aqueous acrylic acid solution was placed into an Erlenmeyer flask of an inner volume of 500ml, 159.3g of 19.3% by weight of an aqueous sodium hydroxide solution was added dropwise under ice-cooling to perform neutralization of 75 mol% of acrylic acid, thereby, 36% by weight of an aqueous acrylic acid partially neutralized salt solution was prepared. To the resulting aqueous acrylic acid partially neutralized salt solution were added 18.4 mg (0.12 mmol) of N,N'-methylenebisacrylamide and 92 mg (0.34 mmol) of potassium persulfate, and this was used as an aqueous monomer solution (b1) for second-stage reverse phase suspension polymerization.

After first-stage reverse phase suspension polymerization was completed, the reaction solution was cooled to room temperature, the aqueous monomer solution (b1) for second-stage polymerization was added dropwise, and the mixture was stirred for 30 minutes. Thereafter, the system was replaced with nitrogen, a temperature was raised to 70 °C, and second-stage reverse phase suspension polymerization was performed.

A total amount of the resulting reaction solution was transferred to a five-neck cylindrical round-bottom flask of an inner volume of 2 liter, equipped with a stirrer, an anchor wing and a condenser, and heated in an oil bath to remove n-heptane, and water under azeotropy with n-heptane, to obtain 468.6g of a hydrous gel of a polymer.

Preparation Example 2

Into an Erlenmeyer flask of an inner volume of 500 ml was placed 92g (1.02 mol) of 80% by weight of an aqueous acrylic acid solution, 152.5g of 20.1% by weight of an aqueous sodium hydroxide solution was added dropwise while ice-cooling to neutralize 75 mol % of acrylic acid, thereby, 37% by weight of an aqueous acrylic acid partially neutralized salt solution was prepared. To the resulting aqueous acrylic acid partially neutralized salt solution were added 18.4 mg (0.11 mmol) of ethylene glycol diglycidyl ether and 92 mg (0.34 mmol) of potassium persulfate, and this was used as an aqueous monomer solution (a2) for first-stage polymerization.

On the other hand, 340 g (500 ml) of n-heptane, and 0.92 g of sucrose fatty acid ester (trade name of Mitsubishi-Kagaku Foods Corporation: S-370) were added to a five-necked cylindrical round-bottom flask of an inner volume of 2 liter, equipped with a stirrer, a two-step paddle wing, a refluxing condenser, an addition funnel and a nitrogen gas introducing tube, to dissolve the material in n-heptane, the aqueous monomer solution (a2) for polymerization was added, and the mixture was suspended under stirring. Thereafter, the system was replaced with nitrogen, a temperature was raised to 70 °C, and first-stage reverse phase suspension polymerization was performed.

Then, separately, 110.4 g (1.23 mol) of 80% by weight of an aqueous acrylic acid solution was placed into an Erlenmeyer flask of an inner volume of 500 ml, 148.1 g of 24.9% by weight of an aqueous sodium hydroxide solution was added dropwise while ice-cooling to neutralize 75 mol % of acrylic acid, thereby, 42% by weight of an aqueous acrylic acid partially neutralized salt solution was prepared. To the resulting aqueous acrylic acid partially neutralized salt solution were added 33.1 mg (0.19 mmol) of ethylene glycol diglycidyl ether and 110 mg (0.41 mmol) of potassium persulfate, and this was used as an aqueous monomer solution (b2) for second-stage reverse phase suspension polymerization.

After first-stage reverse phase suspension polymerization was completed, the reaction solution was cooled to room temperature, the aqueous monomer solution (b2) for second-stage polymerization was added dropwise, and the mixture was stirred for 30 minutes. Thereafter, the system was replaced with nitrogen, a temperature was raised to 70 °C, and second-stage reverse phase suspension polymerization was performed.

The resulting reaction solution was transferred to a five-necked cylindrical round-bottom flask of an inner volume of 2 liter, equipped with a stirrer, an anchor wing, and an condenser, and heated in an oil bath to remove n-heptane, and water under azeotropy with n-heptane, to obtain 469.0 g of a hydrous gel of a polymer.

Preparation Example 3

Into a five-necked cylindrical round-bottom flask of an inner volume of 2 liter, equipped with a stirrer, a two-step paddle wing, a refluxing condenser, an addition funnel and a nitrogen gas introducing tube was placed 184 g (2.04 mol) of 80% by weight of an aqueous acrylic acid solution, 539.7 g of 11.4% by weight of an aqueous sodium hydroxide solution was added dropwise while stirring under cooling to neutralize 75 mol% of acrylic acid, thereby, 25% by weight of an aqueous acrylic acid partially neutralized salt solution was prepared. To this aqueous acrylic acid partially neutralized salt solution were added 36.8 mg (0.24 mmol) of N,N'-methylenebisacrylamide, 184 mg (0.68 mmol) of potassium persulfate and 18.4 mg (0.15 mmol) of sodium sulfite, and a polymerization reaction was performed while retained in a water bath at 40 °C. The resulting polymerization reaction product was ground with a SUS meat chopper to obtain 723.7 g of a hydrous gel of a polymer.

Example 1

Into a flask of an inner volume of 2 liter, equipped with a stirrer, an anchor wing, a condenser and a gas introducing tube, placed 468.6 g of the hydrous gel obtained in Preparation Example 1, and a tip part of the gas introducing tube was adjusted so as to be near a surface of the hydrous gel. Then, a pressure in the flask was retained at 30 kPa with an external pressure-reducing pump (trade name of ULVAC KIKO Inc.: MDA-015) while a nitrogen gas having an oxidation concentration of 3% by volume was ventilated at 200 mL/min through the gas introducing tube, and this was dried at an inner temperature of 80 °C to a water content of a water-absorbing resin of 5.7% to obtain 191.2 g of a water-absorbing resin. A time necessary for drying was 80 minutes.

Example 2

Into a flask of an inner volume of 2 liter, equipped with a stirrer, an anchor wing, a condenser and a gas introducing tube, placed 468.6 g of the hydrous gel obtained as in Preparation Example 1, and a tip part of the gas introducing tube was adjusted so as to be near a surface of the hydrous gel. Then, a pressure in the flask was retained at 65 kPa with an external pressure-reducing pump (trade name of ULVAC KIKO Inc.: MDA-015) while a nitrogen gas having an oxidation concentration of 0.3% by volume was ventilated at 200 mL/min through the gas introducing tube, and this was dried at an inner temperature of 80 °C to a water content of a water-absorbing resin of 8.1% to obtain 195.6 g of a water-absorbing resin. A time necessary for drying was 140 minutes.

Example 3

Into a flask of an inner volume of 2 liter, equipped with a stirrer, an anchor wing, a condenser and a gas introducing tube, placed 468.6 g of the hydrous gel obtained as in Preparation Example 1, and a tip part of the gas introducing tube was adjusted so as to be near a surface of the hydrous gel. Then, a pressure in the flask was retained at 50 kPa with an external pressure-reducing pump (trade name of ULVAC KIKO Inc.: MDA-015) while a nitrogen gas containing no oxygen was ventilated at 200 mL/min through the gas introducing tube, and this was dried at an inner temperature of 95 °C to a water content of a water-absorbing resin of 7.5% to obtain 194.5 g of a water-absorbing resin. A time necessary for drying was 120 minutes.

Example 4

Into a flask of an inner volume of 2 liter, equipped with a stirrer, an anchor wing, a condenser and a gas introducing tube, placed 468.6 g of the hydrous gel obtained as in Preparation Example 1, and a tip part of the gas introducing tube was adjusted so as to be near a surface of a hydrous gel. Then, a pressure in the flask was retained at 50 kPa with an external pressure-reducing pump (trade name of ULVAC KIKO Inc.: MDA-015) while a nitrogen gas having an oxidation concentration of 0.3% by volume was ventilated at 200 mL/min through the gas introducing tube, and this was dried at an inner temperature of 60 °C to a water content of a water-absorbing resin of 7.9% to obtain 195.2 g of a water-absorbing resin. A time necessary for drying was 135 minutes.

Example 5

Into a flask of an inner volume of 2 liter, equipped with a stirrer, an anchor wing, a condenser and a gas introducing tube, placed 468.6 g of the hydrous gel obtained an in Preparation Example 1, and a tip part of the gas introducing tube was adjusted so as to be near a surface of the hydrous gel. Then, a pressure in the flask was retained at 50 kPa with an external pressure-reducing pump (trade name of ULVAC KIKO Inc.: MDA-015) while a nitrogen gas containing no oxygen was ventilated at 200 mL/min through the gas introducing tube, and this was dried at an inner temperature of 70 °C to a water content of a water-absorbing resin of 6.6% to obtain 192.9 g of a water-absorbing resin. A time necessary for drying was 125 minutes.

Example 6

Into a flask of an inner volume of 2 liter, equipped with a stirrer, an anchor wing, a condenser and a gas introducing tube, placed 468.6 g of the hydrous gel obtained as in Preparation Example 1, and a tip part of the gas introducing tube was adjusted so as to be near a surface of the hydrous gel. Then, a pressure in the flask was retained at 40 kPa with an external pressure-reducing pump (trade name of ULVAC KIKO Inc.: MDA-015) while a nitrogen gas containing no oxygen was ventilated at 200 mL/min through the gas introducing tube, and this was dried at an inner temperature of 75 °C to a water content of a water-absorbing resin of 5.0% to obtain 190.0 g of a water-absorbing resin. A time necessary for drying was 100 minutes.

Example 7

Into a flask of an inner volume of 2 liter, equipped with a stirrer, an anchor wing, a condenser and a gas introducing tube, placed 469.0g of the hydrous gel obtained as in Preparation Example 2, and a tip part of the gas introducing tube was adjusted so as to be near a surface of a hydrous gel. Then, a pressure in the flask was retained at 30 kPa with an external pressure-reducing pump (trade name of ULVAC KIKO Inc.:MDA-015) while a nitrogen gas containing no oxygen was ventilated at 200 mL/min through the gas introducing tube, and this was dried at an inner temperature of 80 °C to a water content of a water-absorbing resin of 4.4% to obtain 207.8g of a water-absorbing resin. A time necessary for drying was 80 minutes.

Example 8

Into a flask of an inner volume of 2 liter, equipped with a stirrer, an anchor wing, a condenser and a gas introducing tube, placed 723.7g of the hydrous gel obtained in Preparation Example 3, and a tip part of the gas introducing tube was adjusted so as to be near a surface of the hydrous gel. Then, a pressure in the flask was retained at 30 kPa with an external pressure-reducing pump (trade name of ULVAC KIKO Inc.: MDA-015) while a nitrogen gas containing no oxygen was ventilated at 200 mL/min through the gas introducing tube, and this was dried at an inner temperature of 80 °C to a water content of a water-absorbing resin of 8.3% to obtain 196.0g of a water-absorbing resin. A time necessary for drying was 115 minutes.

Comparative Example 1

Into a flask of an inner volume of 2 liter, equipped with a stirrer, an anchor wing, a condenser and a gas introducing tube, was placed 468.6g of a hydrous gel obtained as in Preparation Example 1, and a tip part of the gas introducing tube was adjusted so as to be near a surface of the hydrous gel. Then, this was dried at an inner temperature of 80 °C under the atmospheric pressure (101.3 kPa) to a water content of a water-absorbing resin of 6.5% while a nitrogen gas having an oxygen concentration of 3% by volume was ventilated at 200 mL/min through the gas introducing tube, to obtain 192.7 g of a water-absorbing resin. A time necessary for drying was 250 minutes.

Comparative Example 2

Into a flask of an inner volume of 2 liter, equipped with a stirrer, an anchor wing, a condenser and a gas introducing tube, was placed 468.6 g of the hydrous gel obtained as in Preparation Example 1, and a tip part of the gas introducing tube was adjusted so as to be near a surface of the hydrous gel. Then, a pressure in the flask was retained at 80 kPa with an external pressure-reducing pump (trade name of ULVAC KIKO Inc.: MDA-015) while a nitrogen gas having an oxygen concentration of 7% by volume was ventilated at 200 mL/min through the gas introducing tube, and this was dried at an inner temperature of 80 °C to a water content of a water-absorbing resin of 9.0% to obtain 197.2 g of a water-absorbing resin. A time necessary for drying was 190 minutes.

Water-absorbing resins obtained in Examples and Comparative Examples were subjected to the following various experiments. Results of the water-absorbing resin performance are shown in Table 1.

(1) Amount of remaining polymerization inhibitor

Two gram of a water-absorbing resin was dispersed in 500 g of a 0.9 wt% physiological saline in a beaker of a volume of 500 mL, and stirred at room temperature for 60 minutes. Then, the dispersion was filtered to separate a water-absorbing resin and a 0.9 wt% physiological saline, and an amount of p-methoxyphenol dissolved in the resulting 0.9 wt% physiological saline was measured by high pressure liquid chromatography under the following conditions.
Column: Shodex RSpak KC-811
Mobile phase: phosphate buffered aqueous solution (pH=2)
Flow rate: 1.2 ml/min
Column temperature: 45 °C
Detector: UV ($\lambda$=210 mn)

(2) Yellow Index

Four gram of the water-absorbing resin was placed into a glass measuring container having an inner diameter of 3 cm and a depth of 1 cm, three stimulation values X, Y and Z were measured using a double beam flicker photometric differential colormeter Z-1001 DP (manufactured by Nippon Denshoku Industries Co., Ltd.), and Yellow Index was calculated by the following equation:

$$\texttt{Yellow Index = 100} \times \texttt{(1.28X-1.062Z)} \div \texttt{Y.}$$

(3) Water-absorption amount

Two gram of a water-absorbing resin was dispersed in 500 g of a 0.9 wt% physiological saline in a beaker of a volume of 500 mL, and the dispersion was stirred for 60 minutes to swell sufficiently. A weight Wa (g) of an opening 75 $\mu$m standard sieve was measured in advance, this was used to filter an aqueous solution containing a swollen gel, and allowed to stand for 30 minutes in the state where the sieve was tilted at a tilt angle of around 30 degree formed relative to a horizontal direction, to remove extra water from the swollen gel. Then, a weight Wb (g) of the sieve containing the swollen gel was measured, and a water absorption amount (g/g) was obtained by the following equation.
Water absorption amount (g/g)=(Wb-Wa)÷2
Table 1

| | Preparation condition | | Water-absorbing resin performance | | |
|---|---|---|---|---|---|
| | Oxygen concentration (vol%) | Pressure (kPa) | Remaining polymerization inhibitor (ppm) | Yellow Index | Water absorption amount (g/g) |
| Example 1 | 3 | 30 | 16 | 17 | 66 |

(continued)

| | Preparation condition | | Water-absorbing resin performance | | |
|---|---|---|---|---|---|
| | Oxygen concentration (vol%) | Pressure (kPa) | Remaining polymerization inhibitor (ppm) | Yellow Index | Water absorption amount (g/g) |
| Example 2 | 0.3 | 65 | 22 | 13 | 67 |
| Example 3 | 0 | 50 | 17 | 12 | 63 |
| Example 4 | 0.3 | 50 | 20 | 13 | 66 |
| Example 5 | 0 | 50 | 17 | 10 | 64 |
| Example 6 | 0 | 40 | 14 | 10 | 64 |
| Example 7 | 0 | 30 | 10 | 9 | 62 |
| Example 8 | 0 | 30 | 14 | 13 | 68 |
| Comparative Example 1 | 3 | 101.3 | 43 | 32 | 81 |
| Comparative Example 2 | 7 | 80 | 31 | 48 | 88 |

As apparent from Table 1, since the water-absorbing resin obtained in each Example is reduced in a remaining polymerization inhibitor, coloring is suppressed to Yellow Index of 20 or less. On the other hand, the water-absorbing resin obtained in each Comparative Example is not reduced in a remaining polymerization inhibitor, and has Yellow Index exceeding 20, and it is seen that the resin is clearly colored even visually.

Industrial applicability

Since the water-absorbing resin obtained by the process of the present invention is small in a remaining polymerization inhibitor, and is colored little, the resin is suitably used, particularly, in a hygiene material such as a sanitary product, a paper diaper and the like.

**Claims**

1.   A process for producing a water-absorbing resin, comprising polymerizing a water-soluble ethylenic unsaturated monomer and, then, drying a hydrous gel of the resulting polymer at a pressure of 5 to 90 kPa under the atmosphere having no oxygen, or having an oxygen concentration of not higher than 5% by volume.

2.   The process for producing a water-absorbing resin according to claim 1, wherein drying is performed at 60 to 110 °C.

3.   A water-absorbing resin having an amount of a remaining polymerization inhibitor of not more than 30 ppm, obtained by the process as defined in claim 1 or 2.

4.   A water-absorbing resin having Yellow Index of not more than 20, obtained by the process as defined in claim 1 or 2.

## INTERNATIONAL SEARCH REPORT

| | International application No. |
|---|---|
| | PCT/JP2005/011440 |

A. CLASSIFICATION OF SUBJECT MATTER
    Int.Cl$^7$  C08F6/00

According to International Patent Classification (IPC) or to both national classification and IPC

B. FIELDS SEARCHED

Minimum documentation searched (classification system followed by classification symbols)
    Int.Cl$^7$  C08F6/00-6/28

Documentation searched other than minimum documentation to the extent that such documents are included in the fields searched
    Jitsuyo Shinan Koho            1922-1996    Jitsuyo Shinan Toroku Koho    1996-2005
    Kokai Jitsuyo Shinan Koho      1971-2005    Toroku Jitsuyo Shinan Koho    1994-2005

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2000-26510 A  (Sanyo Chemical Industries, Ltd.), 25 January, 2000 (25.01.00), Claims 1, 20, 29, 43; Par. Nos. [0031], [0032] (Family: none) | 1-4 |
| Y | JP 7-278224 A  (Nippon Shokubai Kagaku Kogyo Co., Ltd.), 24 October, 1995 (24.10.95), Claims 1 to 3, 8; Par. Nos. [0009], [0032] (Family: none) | 1-4 |

☒ Further documents are listed in the continuation of Box C.          ☐ See patent family annex.

| | | |
|---|---|---|
| * | Special categories of cited documents: | |
| "A" | document defining the general state of the art which is not considered to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search 09 September, 2005 (09.09.05) | Date of mailing of the international search report 27 September, 2005 (27.09.05) |
|---|---|
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.

PCT/JP2005/011440

C (Continuation).   DOCUMENTS CONSIDERED TO BE RELEVANT

| Category* | Citation of document, with indication, where appropriate, of the relevant passages | Relevant to claim No. |
|---|---|---|
| X<br>Y | JP 11-322846 A   (Nippon Shokubai Kagaku Kogyo Co., Ltd.),<br>26 November, 1999 (26.11.99),<br>Claims 1, 7, 9; Par. Nos. [0048], [0128] to [0132]<br>& US 6444744 B1          & EP 942014 A2<br>& CN 1234407 A          & BR 9900992 A<br>& SG 75923 A | 3,4<br>1,2 |
| X | JP 2003-246810 A   (Nippon Shokubai Kagaku Kogyo Co., Ltd.),<br>05 September, 2003 (05.09.03),<br>Claims 1, 9; Par. Nos. [0095], [0101]<br>& US 2004110914 A1        & EP 1456259 A<br>& WO 03/51940 A1         & BR 206696 A | 3,4 |
| X | JP 2003-206305 A   (Sumitomo Seika Chemicals Co., Ltd.),<br>22 July, 2003 (22.07.03),<br>Claims 1, 5; Par. Nos. [0045], [0082]<br>& US 200585604 A1        & EP 1466928 A1<br>& WO 03/59962 A1 | 4 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003048915 A